# EUROPEAN PATENT SPECIFICATION

(11) **EP 0 435 860 B1**
(45) Date of publication and mention of the grant of the patent: **25.08.1993**
(21) Application number: 91200323.3
(22) Date of filing: 28.05.1987
(51) Int. Cl.: A61M 5/14

(54) **Adapter for connecting with a drug vial to introduce a beneficial agent to a patient**
Adapter zur Verbindung mit einer Ampulle zur Verabreichung eines Heilmittels
Adapteur pour connecter un flacon pour administrer un remède à un patient

(30) Priority: 29.05.1986 US 868827
(43) Date of publication of application: 03.07.1991
(62) Divisional of application: 87903792.7
(73) Proprietor: BAXTER INTERNATIONAL INC., Deerfield, IL 60015 (US)
(72) Inventor: Zdeb, Brian D., Round Lake Park, Illinois 60073 (US); Slater, Glenn L., Ogden Utah 84403 (US)
(74) Representative: MacGregor, Gordon

(56) References cited:
- EP-A- 0 077 604
- WO-A-86/03147
- US-A- 2 955 595

## Description

### Technical Field of the Invention

The present invention is related to the delivery of a beneficial agent to a patient and is more particularly directed to the passive delivery of the beneficial agent to the intravenous system of a patient in a safe and effective manner.

### Background of the Invention

Many drugs are mixed with a diluent before being delivered intravenously to a patient. The diluent may be, for example, a dextrose solution, a saline solution or even water. Many such drugs are supplied in powder form and packaged in glass vials or ampules. Other drugs, such as some used in chemotherapy, are packaged in glass vials or ampules in a liquid state.

Powdered drugs may be reconstituted in a well known manner, utilizing a syringe which is used to inject liquid into the vial for mixing, the syringe eventually withdrawing the mixed solution from the vial. When a drug must be diluted before delivery to a patient the drug is often injected into a container of diluent after it is reconstituted, where the container may be connected to an administration set for delivery to a patient. More specifically, the diluent is often packaged in glass bottles, or flexible plastic containers such as are sold under the names MINI-BAG™ AND VIAFLEX® by Travenol Laboratories, Inc. of Deerfield, Illinois. These containers have administration ports for connection to an administration set which delivers the container contents from the container to the patient. The drug is typically added to the container through an injection site on the container.

Drugs may be packaged separately from the diluent for various reasons. One of the most important reasons is that many drugs do not retain their chemical and physical stability when mixed with a diluent and thus cannot be stored for any substantial period of time. Also, drugs are often packaged separately from the diluent because many firms which manufacture drugs are not engaged in the business of providing medical fluids in containers for intravenous delivery, and vice versa.

Therefore, a doctor, nurse, pharmacist or other medical personnel must mix the drug and diluent. This presents a number of problems. The reconstitution procedure is time consuming and requires aseptic technique. The operator must provide the proper diluent and a syringe before beginning. Often the powdered drug is "caked" at the bottom of the vial. Thus, when liquid is injected into the vial from a syringe the surface area of contact between the liquid and the powdered drug may be quite small initially, thus making the mixing procedure even more time consuming. Because of the limited vial volume, the increasing drug concentration in the diluent makes it harder to finish the reconstitution process. The operator may attempt to solve this by repeatedly injecting solution into the vial, mixing and withdrawing the solution but this makes necessary additional injections and movement of the syringe which increase the likelihood of contamination. Also, it is sometimes difficult to get all of the drug and/or liquid out of the vial, thus increasing the time required to perform the reconstitution procedure.

The reconstitution procedure should be performed under preferably sterile conditions. In addition to such a requirement making the operator justifiably more cautious and consuming more time, sterile conditions are often hard to maintain. In some instances, a laminar flow hood may be required under which the reconstitution procedure is performed.

Some drugs, such as some chemotherapy drugs, are toxic. Exposure of the operator to the drugs during reconstitution may be dangerous, especially if the operator works with such drugs on a daily basis and is repeatedly exposed to them.

A further problem is that the reconstitution procedure provides a source of confusion as to which container contains which drug. The diluent container should be marked with the drug with which it has been injected and the name of the patient to whom it should be delivered.

After a drug is reconstituted and withdrawn into a syringe barrel, the drug may in some instances be injected immediately into the intravenous system of a patient. More typically however, the reconstituted drug is injected from the syringe into a larger container of solution as discussed above, for connection to an intravenous administration set. This is because often the drug reconstituted in the syringe is still at a concentration so high as to cause local toxicity in the veins of a patient near the injection site where the needle pierces the skin. This may create severe vein irritation which may be medically harmful. Additionally, even though the proper dose of medication is in the syringe, immediate injection into the patient's blood stream may create a condition of systemic toxicity wherein the level of drug concentration in the patient's entire blood stream is dangerously high. Yet another reason for not making the injection from the syringe directly into the patient is that it creates an additional injection site into the patient, which may be painful for the patient and provides another opportunity for infection.

For these reasons, the reconstituted drug is more typically injected into a diluent container.

A patient may typically be administered a dextrose or saline solution from a large volume parenteral container, for example, such as a one liter container, delivered through an administration set such as a CONTINU-FLO® administration set sold by Travenol Laboratories. If the reconstituted drug were injected into the large volume parenteral container, delivery of the drug would usually be delivered over too long a time period. Often, these large volume fluids are delivered at very slow flow rates.

More typically, the reconstituted drug is injected into a small volume parenteral container, such as a fifty milliliter container sold by Travenol Laboratories. This MINIBAG™ container is hung at a higher elevation than the large volume parenteral container and is connected by a secondary administration set to an injection site on the primary administration set. Because it is maintained at a higher elevation, the reconstituted drug in the small volume container is delivered, after which fluid from the large volume container begins to flow once more. By utilizing a small volume container connected to an administration set for delivery of the drug or other beneficial agent instead of a direct syringe injection, the drug is delivered over a preferred time period that tends to minimize negative side effects.

A closed reconstitution delivery system is disclosed in U.S. Patent Nos. 4,410,321; 4,411,662; 4,432,755; and 4,458,733, all assigned to Baxter Travenol Laboratories Inc., the assignee of the present invention. As shown therein, a container includes a drug and a diluent in separate compartments which are reconstituted in a closed system before the drug is delivered to the patient. Typically, the container is connected to an administration set which is connected at its other end to the primary administration set, such as with the small volume parenteral container described above. The container shown in these patents solves many of the problems associated with syringe reconstitution. The product does however necessitate a series of reconstitution steps which must be performed by the nurse or other operator prior to delivering the fluid from the container.

Delivery of a drug or other beneficial agent in a manner not requiring reconstitution steps by an operator is shown in U.S. Patent Nos. 4,424,056; 4,432,756; 4,439,183; 4,474,574; 4,479,793; 4,479,794; 4,525,162, and 4,548,599 and Canadian Patent No. 1,173,795, assigned to Alza Corporation of Palo Alto, California. As disclosed in those patents, a parenteral delivery system is disclosed which has a formulation chamber therein for administering a beneficial agent such as a drug. The system is advantageous in that it provides for reconstitution of the drug by fluid flowing from a large volume parenteral container for example, through the administration set containing the formulation chamber with the drug therein. The system intends to eliminate the need for the time consuming reconstitution procedure described above and appears to eliminate the problems associated with the reconstitution procedure.

Another passive reconstitution system is disclosed in European Patent Application No. 0059694 to Aktiebolaget Hassle of Sweden.

Still another device for delivering a drug "in-line", i.e., in the administration set, is disclosed in U.S. Patent No.4,534,757 assigned to Alza Corporation. The device holds the drug and includes a section through which the liquid passes in a direction substantially opposite to the general direction in which liquid flows to the patient.

Yet another system which attempts to provide for drug reconstitution in-line without manual reconstitution by a nurse or other operator is shown in U.S. Patent No. 4,465,471, assigned to Eli Lilly and Co. of Indianapolis, Indiana. That patent discloses constructions for a receptacle in the administration set itself. A separate cartridge containing the drug to be reconstituted and delivered to the patient is plugged into the receptacle. As liquid enters the cartridge for reconstitution of the drug and subsequent delivery out of the cartridge and receptacle and into the patient, some or most fluid continues to flow through the administration set, bypassing the cartridge entirely.

European Patent Application Publication No. 0146310 to Eli Lilly and Co. is directed to a system for drug reconstitution including an intravenous administration set and a drug vial and utilizes the vial vacuum to reconstitute the drug.

U.S. Patent No. 4,534,758 to Akers et al. discloses a relatively complex drug delivery apparatus with various valves. When liquid from a container is delivered to the drug vial, the vial is to be agitated for a time sufficient to suspend the previously dry medicine.

U.S. Patent No. 4,581,014 to Millerd et al. assigned to Ivac Corporation of San Diego, California discloses a selector valve for delivering a previously reconstituted drug from a drug vial through an intravenous administration set to a patient.

All the publications described above are directed to solutions to the time consuming reconstitution procedure and/or its associated problems, such as delivery of the solution to a patient. In most of the offered solutions, delivery of the drug is intended to be passive, i.e., once the drug is placed into the administration set, manual reconstitution steps are not required.

Still another common feature of many of the attempted solutions disclosed in these publications is that delivery of the drug is intended to be able to be made in a manner which is essentially independent of the fluid flow rate through the administration set and into the patient. Stated differently, some of the systems are designed to deliver a certain dosage of drug in a preselected time period, within a broad range of fluid flow rates. Delivery of a drug independent of flow rate is desirable because it ensures that the necessary dosage will be delivered within a therapeutically acceptable time period, which may be typically about twenty to thirty minutes, although this time period may vary depending upon the drug and dosage.

By making delivery of the drug or other beneficial agent independent of the flow rate, the system ensures that the drug will not be delivered too quickly should the flow rate be set too high by the nurse or other operator, thereby preventing the problem of systemic toxicity discussed above.

Some of the documents, such as U.S. Patent Nos. 4,424,056; 4,479,793; and 4,479,794, are also directed to systems having a beneficial agent placed "in-line" in an administration set for mixing of the agent and delivery to a patient, wherein the delivery of the agent may be made in a given volume of fluid. Also, a valve controlling fluid flow may be manually operated to deliver the agent in a manner which can be made dependent upon fluid flow.

At least the automatic reconstitution type systems discussed above, (i.e., those not requiring a separate agitation or mixing step), suffer from the possibility of creating a concentration of beneficial agent in the fluid being delivered to the patient which is too high at low flow rates. This results in local toxicity to the patient near the point of introduction into the body. The problem is solved by the invention disclosed in WO-A-86/03417, which was published after the priority date of Claim 1. Further solutions to the problems of passively mixing and delivering a beneficial agent to a patient are disclosed in WO-A-86/03416, which also was published after the priority date of Claim 1. In that application there is disclosed certain housing constructions for delivering the beneficial agent to the patient. Typically, the housing includes a receptable which is placed in-line in a medical liquid administration set and a separate cartridge including the beneficial agent. The cartridge is plugged into the receptable when it is desired to deliver the beneficial agent to the patient. Active reconstruction by a nurse or other operator is not required. Instead, once the cartridge is plugged into the receptable, liquid flowing from the source of medical liquid through the administration set flows into the receptacle and the agent-containing cartridge, reconstituting the agent. The solution with agent therein flows out the receptacle, down the administration set to the patient's venous system.

The present invention provides an adapter adapted to connect a drug vial having a pierceable stopper and containing a beneficial agent, with a receptacle (42) mounted in a fluid conduit and having a pierceable situs (80), so that the adapter provides for selective fluid communication between the vial and the receptacle to introduce beneficial agent into the fluid conduit for delivery of the beneficial agent to a patient, the beneficial agent being passively reconstituted during fluid flow through the fluid conduit, said adapter and the drug vial, said adapter comprising:
(a) a hollow, rigid shell including an enlarged end portion for connecting to the drug vial, said shell being adapted for mounting about the receptacle, ;
(b) a slidable plate, slidably mounted within said hollow rigid shell and including inlet flow path means and outlet flow path means mounted to said slidable plate, said flow path means further including stopper piercing means and situs piercing means;
   wherein movement of said slidable plate enables sliding said flow path means towards said enlarged end portion for piercing the stopper in use, for placing the drug vial the inlet flow path means and said outlet flow path means in open communication, with the outlet flow path means extending further into the enlarged portion than the inlet flow path means.

### Description of the Drawings

Fig.1 is a perspective view of an administration set including an air flask and a receptacle;
Fig.2 is an enlarged, fragmentary, cross-sectional view of the receptacle illustrated in Figure 1;
Fig.3 is a fragmentary, enlarged, cross-sectional view of the air flask illustrated in Figure 1.
Fig.4 is a sectional view of an adapter according to the invention together with a drug vial and receptacle; and
Fig.5 is a similar view of an alternative embodiment of an adapter.

### Detailed Description

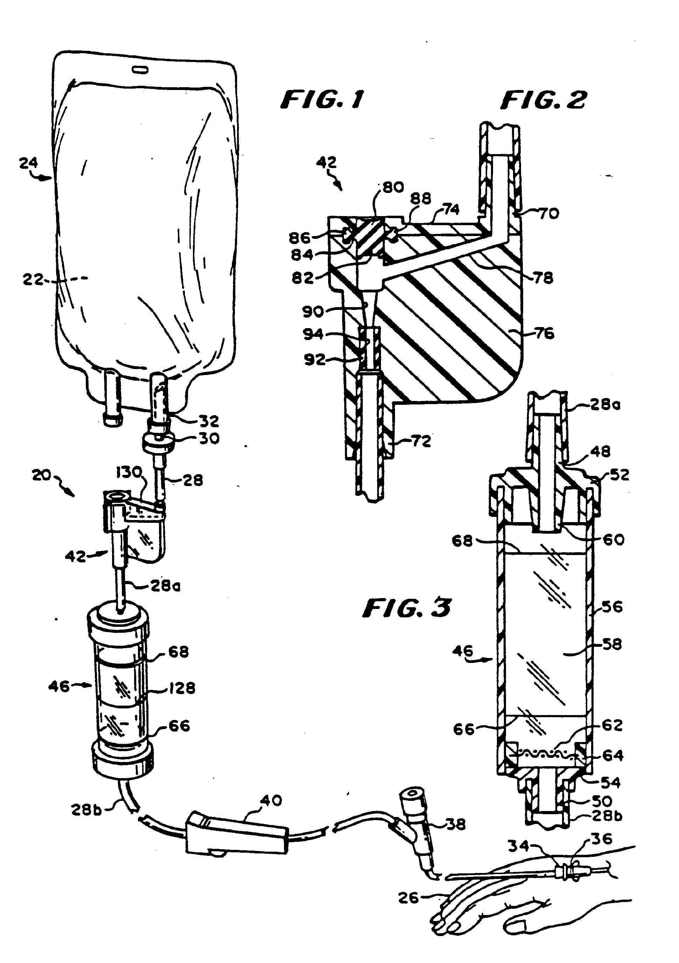

Referring to Fig. 1, there is illustrated an administration set 20 for the delivery of a medical liquid, stored within a medical liquid source such as large volume parenteral container 24, to a patient 26. The administration set 20 includes a fluid conduit 28 made for example of flexible polyvinylchloride tubing. Upstream connection means such as a standard intravenous administration set spike 30 is mounted at the upstream end of the fluid conduit 28. The spike is adapted for piercing the membrane of the container administration port 32.

The fluid conduit 28 includes downstream connection means such as a Luer taper 34 mounted at the downstream end of the fluid conduit 28. The Luer taper 34 may be connected in accordance with standard technique to a venous catheter 36.

The administration set 20 may further include a standard pierceable injection site 38 for injecting a medical liquid by means of a needle through the injection site 38. The administration set 20 may further include flow rate control means such as a standard roller clamp 40 mounted about the flow conduit 28.

The administration set 20 further includes a unique receptacle 42 shown in greater detail in Fig. 2. The receptacle 42 is an improvement to the receptacle disclosed in WO-A-8603417. The receptacle 42 is mounted along the fluid conduit and is adapted for receiving an adaptor 160 and drug vial 162 containing beneficial agent. When the adaptor and vial are mounted upon the receptacle, at least some, and preferably all liquid from the medical liquid source container 24 that flows through the fluid conduit 28 into the receptacle 42 also flows through the vial 162 before passing downstream out of the receptacle to the patient.

Downstream of the receptacle 42 is an air chamber 46, illustrated in Figs. 1 and 3. As will be explained in greater detail below, the air flask 46 permits automatic priming of the cartridge 44 upon mounting of the cartridge on the receptacle 42 of the administration set 20. The air flask 46 absorbs the air disposed within the cartridge 44 and prevents that air from passing downstream to the patient.

Referring to Fig. 3, the air flask 46 includes an inlet 48 mounted to and receiving fluid from upstream fluid conduit 28a. The air flask 46 includes an outlet 50 mounted to and transferring liquid to downstream fluid conduit 28b. The inlet and outlet may be mounted to the fluid conduit 28 by means of interference fit, solvent bonding etc. The air flask 46 is mounted downstream of the receptacle 42.

In the preferred embodiment the air flask 42 includes inlet and outlet end caps 52, 54 respectively between which is mounted a cylindrical sidewall 56 of preferably optically transparent, flexible material such as polyvinyl chloride . The sidewall 56 and the end raps 52, 54 define an air chamber 58 having a cross-sectional diameter that is greater than the internal diameter of the fluid conduit 28, so that liquid entering the air chamber 58 from the drop forming orifice 60 adjacent the inlet 48 falls toward the outlet 50. The air flask 46 thus provides a collection reservoir for air within the administration set 20.

The air flask 46 further includes particulate matter barrier means such as a particulate matter screen 62 mounted within a plastic ring 64 near the outlet 50. The particulate matter barrier may in fact be a sterilizing filter having a nominal pore size of about 0.2 micron. The nominal pore size may be much larger, such as a gross particulate matter barrier having a nominal pore size of about 20 microns. In the preferred embodiment the nominal pore size is about 10 microns. The screen may be a nylon mesh material such as supplied by filter Tek of Hebron, Illinois. The particulate matter barrier 62 is mounted transverse to the fluid path such that all liquid passing through the air flask 46 must pass through the particulate matter barrier 62.

The particulate matter barrier 62 need not be disposed within the air flask 46 but the barrier should be mounted downstream of the receptacle 42 so that all liquid that exits the inserted cartridge 44 will pass through the particulate matter barrier. Also, it is possible to construct the receptacle 42, the air flask 46 and the particulate matter barrier 62 as a single unit rather than as separated by upstream fluid conduit 28a for example.

In the preferred embodiment, the air flask 46 includes a minimum liquid level indicator 66 and a maximum liquid level indicator 68 which may for example comprise lines around the periphery of the air flask 46. The liquid level in the air flask 46 should preferably be somewhere between the minimum and maximum liquid level indicators 66, 68 immediately before insertion of the cartridge 44 within the receptacle 42.

The improved receptacle 42 includes a receptacle inlet 70 and a receptacle outlet 72 connected to the fluid conduit 28. The air flask 46 is disposed downstream of the receptacle outlet 72.

The receptacle 42 includes upper and lower fitments 74, 76 respectively. The upper fitment 74 includes the inlet. The lower fitment 76 includes the outlet 72 and a fluid receiving segment 78 having an upstream end in fluid communication with the inlet 70 and a downstream end in fluid communication with the outlet 72.

Pierceable situs 80 is mounted within the receptacle 42, trapped between the upper and lower fitments 74, 76. The pierceable situs 80 includes a pierceable main body portion 82 and a ring-like extension 84 extending about the periphery of the main body portion 82. The ring-like extension 84 further includes an enlarged outer periphery 86.

Together, the upper and lower fitments 74, 76 define an annular channel 88 substantially corresponding to and receiving the ring-like extension 84 including the enlarged periphery 86 thereof, such that the upper and lower fitments trap the pierceable situs 80 therebetween in secure fashion. The situs 80 may not be removed without destruction of the receptacle 42. The upper and lower fitments 74, 76 may be bonded together by adhesive, ultrasonic sealing etc. It is important that the situs be securely maintained within the receptacle because a plurality of adaptors 160, each having two piercing cannulas, may be repeatedly inserted and withdrawn from the situs during the useful life of the receptacle 42 and administration set 20. The fluid receiving segment 78 includes a tapered portion 90 below and generally coaxial with the pierceable situs 80. The tapered portion 90 serves as a needle guide into the resilient bushing 92.

The resilient bushing is preferably made of an elastomer such as polyisoprene. The resilient bushing 92 defines a narrow through-bore 94. The resilient bushing is juxtaposed relative to the pierceable situs 80 so that the through-bore 94 is substantially coaxial with the tapered portion 90.

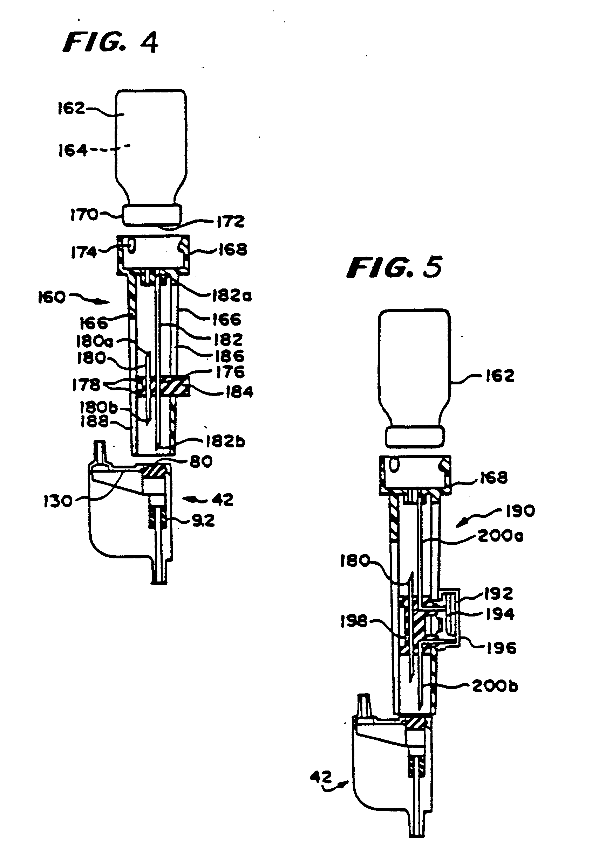

There is illustrated in Fig. 4 an adapter 160 for connecting a chamber such as a standard drug vial 162 having a beneficial agent 164 therein to a receptacle 42. The adapter 160 includes a hollow rigid shell 166 with an enlarged vial end 168 for a snap fit engagement with the mouth 170 of the vial 162. The vial 162 includes a pierceable rubber stopper 172 therein. The enlarged vial end 168 may include projections 174. A reconstitution device showing a similar snap fit arrangement is disclosed in WO-A-8601487. The adapter 160 includes a sliding plate 176 slidably mounted within the hollow rigid shell 166. The sliding plate 176 may be of a preferably resilient material which may be slid along the longitudinal wall of the shell 166. The sliding plate 176 may include projections 178 slidably received within recesses in the shell wall. The resilient material and the projections 178 are intended to keep the sliding plate 176 stationary until movement is intended.

Mounted within the sliding plate 176 is a first hollow cannula 180 having a first pointed hollow end 180a facing the enlarged vial end 168 and a hollow pointed end 180b facing opposite the enlarged end 168. Also mounted within the sliding plate 176 is a second hollow cannula 182 including a hollow pointed first end 182a facing the enlarged end 168 and a second hollow pointed end 182b facing opposite the enlarged end 168. The sliding plate 176 includes a handle portion 184 projecting out of the shell 166 at a handle receiving slot 186 within the shell wall. The rigid shell 166 also includes a receptacle-receiving slot 188 for mounting about the bridge 130 of the receptacle 42.

The first hollow cannula 180 comprises an inlet flow path means into the drug vial 162 or other chamber. The second hollow cannula 182 comprises a separate outlet flow path out of the drug vial 162. The first ends 180a and 182a of the cannulas 180, 182 comprise chamber piercing means for piercing the rubber stopper 172 of the drug vial 162. The second ends 180b, 182b of the cannulas comprise receptacle piercing means.

In operation, the nurse or other medical personnel snaps the drug vial 162 within the enlarged end portion 168 of the adapter 160. The operator then grabs the handle portion 184 and moves it within the slot 186, thereby sliding the sliding plate 176 and the needles mounted therein toward the drug vial 162, piercing the rubber stopper 172 with both the first and second cannulas 180, 182. The adapter 160 is then mounted about the receptacle 42 with the shell 166 fitting thereabout and with the first and second cannulas 180, 182 piercing the pierceable situs 80, with the second cannula 182 engaging the bushing 92.

Referring now to Fig. 5, there is shown an alternate embodiment of the adapter 190 similar to the adapter 160 shown in Fig. 12A. Here however the handle portion 196 that extends from the sliding plate 198 includes an air vent 192 such as a bacteria blocking hydrophobic membrane and a sterilizing .22 micron membrane filter 194. The second hollow cannula 200 is formed by two separate segments, segment 200a facing the enlarged adapter end portion 168 and segment 200b facing away from the adapter end portion 168. The segments 200a, 200b are in open communication through the interior of the handle portion 196, across the filter 194. Operation of the adapter 190 is the same as operation of the adapter 160, except that inclusion of the air vent 192 provides an exhaust for air within the drug vial 162 during priming. Also, the particulate matter barrier 194 is mounted within the adapter 190, preventing particulate matter from going downstream to the patient.

## Claims

1. An adapter adapted to connect a drug vial (162), having a pierceable stopper (172) and containing a beneficial agent (164), with a receptacle (42) mounted in a fluid conduit and having a pierceable situs (80), so that the adapter provides for selective fluid communication between the vial and the receptacle to introduce beneficial agent into the fluid conduit for delivery of the beneficial agent to a patient, the beneficial agent being passively reconstituted during fluid flow through the fluid conduit, said adapter and the drug vial, said adapter comprising:
(a) a hollow, rigid shell (166) including an enlarged end portion (168) for connecting to the drug vial (162), said shell being adapted for mounting about the receptacle (42),;
(b) a slidable plate (176) slidably mounted within said hollow rigid shell (166) and including inlet flow path means (180) and outlet flow path means (182) mounted to said slidable plate, said flow path means further including stopper piercing means (180a,182a) and situs piercing means (180b,182b);
wherein movement of said slidable plate enables sliding said flow path means (180,182) towards said enlarged end portion (168) for piercing the stopper (172) in use, for placing the drug vial (162), the inlet flow path means (180) and said outlet flow path means (182) in open communication, with the outlet flow path means (182) extending further into the enlarged portion (168) than the inlet flow path means.

2. An adapter according to Claim 1, wherein the slidable plate (176) is made of resilient material.

3. An adaptor according to Claim 1 or 2, wherein the slidable plate (176) has a projection (178) slidably received in a recess in the wall of the shell (166).

4. An adapter according to Claim 1, 2 or 3, wherein each of the piercing means (180a,182a) comprises a pointed end of a cannula (180,182) defining the respective flow path means.

5. An adapter according to any preceding claim, wherein the slidable plate (176) includes a handle portion (184) projecting out from the shell (166).

6. An adapter according to Claim 5, wherein the handle portion (184) includes a bacteria blocking air vent (192) communicating with the outlet flow path means (182).

7. An adapter according to Claim 6, wherein the air vent includes a bacteria blocking filter (194).

8. An adapter according to any preceding claim, including a drug vial (162) with a pierceable stopper (172) having a mouth (170) snap-engageable in the enlarged end portion (168) of the shell (166).

## Patentansprüche

1. Adapter zur Verbindung eines Arzneimittelfläschchens (162), das einen durchstoßbaren Verschluß (172) aufweist und ein wohltuendes Mittel (164) enthält, mit einer Aufnahme (42), die in einer Fluidleitung angebracht ist und eine durchstoßbare Stelle (80) besitzt, in einer derartigen Weise, daß der Adapter eine selektive Fluidverbindung zwischen dem Fläschchen und der Aufnahme zum Einleiten von wohltuendem Mittel in die Fluidleitung zur Zuführung des wohltuenden Mittels zu einem Patienten bildet, wobei das wohltuende Mittel während der Fluidströmung durch die Fluidleitung, den Adapter und das Arzneimittelfläschchen passiv aufbereitet wird, wobei der Adapter folgendes aufweist:
(a) einen hohlen starren Mantel (166) mit einem vergrößerten Endbereich (168) zur Verbindung mit dem Arzneimittelfläschchen (162), wobei der Mantel um die Aufnahme (42) herum anbringbar ist;
(b) eine Gleitplatte (176), die innerhalb des hohlen starren Mantels (166) gleitend verschiebbar angebracht ist und eine Eintrittsströmungswegeinrichtung (180) und eine Austrittsströmungswegeinrichtung (182) aufweist, die an der Gleitplatte angebracht sind, wobei die Strömungswegeinrichtungen weiterhin Einrichtungen (180a, 182a) zum Durchstoßen des Verschlusses sowie Einrichtungen (180b, 182b) zum Durchstoßen der durchstoßbaren Stelle aufweisen;
wobei eine Bewegung der Gleitplatte eine Gleitbewegung der Strömungswegeinrichtungen (180, 182) in Richtung auf den vergrößerten Endbereich (168) zum Durchstoßen des Verschlusses (172) im Gebrauch ermöglicht, um dadurch das Arzneimittelfläschchen (162), die Eintrittsströmungswegeinrichtung (180) und die Austrittsströmungswegeinrichtung (182) in offene Verbindung zu bringen, wobei sich die Austrittsströmungswegeinrichtung (180) weiter in den vergrößerten Bereich (168) hineinerstreckt als die Eintrittsströmungswegeinrichtung.

2. Adapter nach Anspruch 1,
bei dem die Gleitplatte (176) aus einem elastischen Material gebildet ist.

3. Adapter nach Anspruch 1 oder 2,
bei dem die Gleitplatte (176) einen Vorsprung (178) aufweist, der in einer Aussparung in der Wand des Mantels (166) gleitend verschiebbar aufgenommen ist.

4. Adapter nach Anspruch 1, 2 oder 3,
bei dem jede Durchstoßeinrichtung (180a, 182a) ein spitzes Ende einer die jeweilige Strömungswegeinrichtung bildenden Kanüle (180, 182) aufweist.

5. Adapter nach einem der vorhergehenden Ansprüche,
bei dem die Gleitplatte (176) einen aus dem Mantel (166) herausragenden Handhabungsbereich (184) aufweist.

6. Adapter nach Anspruch 5,
bei dem der Handhabungsbereich (184) eine mit der Austrittsströmungswegeinrichtung (182) kommunizierende, Bakterien blockierende Entlüftungseinrichtung (192) aufweist.

7. Adapter nach Anspruch 6,
bei dem die Entlüftungseinrichtung ein Bakterien blockierendes Filter (194) aufweist.

8. Adapter nach einem der vorausgehenden Ansprüche,
mit einem Arzneimittelfläschchen (162), das einen durchstoßbaren Verschluß (172) mit einem Mundstück (170) aufweist, das in dem vergrößerten Endbereich (168) des Mantels (166) mit einem Schnappeingriff aufnehmbar ist.

## Revendications

1. Adaptateur conçu pour connecter une fiole à médicament (162) ayant un bouchon perçable (172) et contenant un agent avantageux (161) avec un réceptacle (42) monté dans une conduite de liquide et ayant un emplacement perçable (80) de telle sorte que l'adaptateur permette une communication sélective de liquide entre la fiole et le réceptacle afin d'introduire l'agent avantageux dans la conduite pour liquide en vue de l'administration de l'agent avantageux à un patient, l'agent avantageux étant reconstitué passivement pendant l'écoulement du liquide à travers la conduite de liquide, ledit adaptateur et ladite fiole à médicament, ledit adaptateur comprenant :
(a) une coque rigide et creuse (166) comprenant une partie terminale élargie (168) pour la connexion de la fiole de médicament (162), ladite coque étant disposée pour être montée sur le réceptacle (42);
(b) une plaque coulissante (176) montée à coulisse dans ladite coque rigide creuse (166) et comprenant un passage d'écoulement d'entrée (180) et un passage d'écoulement de sortie (182) montés dans ladite plaque coulissante, ledit passage d'écoulement (180a, 182a) comprenant en outre un moyen de perçage de bouchon et un moyen de perçage d'emplacement (180b, 182b);
dans lequel le mouvement de ladite plaque coulissante permet de faire coulisser ledit trajet d'écoulement (180, 182) vers ladite partie terminale élargie (168) pour percer le bouchon (172) utilisé pour placer la fiole à médicament (162), le trajet d'écoulement d'entrée (180) et le trajet d'écoulement de sortie (182) en communication ouverte avec le trajet d'écoulement de sortie (182) s'étendant plus loin dans la partie élargie (168) que le trajet d'écoulement d'entrée.

2. Adaptateur selon la revendication 1, dans lequel la plaque coulissante (176) est réalisée en un matériau élastique.

3. Adaptateur selon la revendication 1 ou 2, dans lequel la plaque coulissante (176) a un élément en saillie (178) monté à coulisse dans un redan de la paroi de la coque (166).

4. Adaptateur selon la revendication 1, 2 ou 3, dans lequel chacun des moyens de perçage (180a, 182a) comprend une extrémité pointue d'une canule (180, 182) définissant les trajets d'écoulement respectifs.

5. Adaptateur selon l'une quelconque des revendications précédentes, dans lequel la plaque coulissante (176) comprend une partie de poignée (184) faisant saillie hors de la coque (166).

6. Adaptateur selon la revendication 5, dans lequel la partie de poignée (184) comprend un évent pour air arrêtant les bactéries (192) en communication avec le trajet d'écoulement de sortie (182).

7. Adaptateur selon la revendication 6, dans lequel l'évent pour air comprend un filtre arrêtant les bactéries (194).

8. Adaptateur selon l'une quelconque des revendications précédentes, comprenant une fiole à médicament (162) avec un bouchon perçable (172) ayant une embouchure (170) pouvant venir s'emboîter dans la partie terminale élargie (168) de la coque (166).
